(19) Europäisches Patentamt
European Patent Office
Office européen des brevets

(11) Veröffentlichungsnummer: **0 172 513 B1**

(12) # EUROPÄISCHE PATENTSCHRIFT

(45) Veröffentlichungstag der Patentschrift: **24.07.91**

(51) Int. Cl.5: **A61K 6/08**, A61K 6/06

(21) Anmeldenummer: **85110085.9**

(22) Anmeldetag: **12.08.85**

(54) **Verwendung von porösen Füllstoffen in polymerisierbaren Dentalmassen, solche Massen und deren Verwendung zur Herstellung von Formkörpern.**

(30) Priorität: **22.08.84 DE 3430801**

(43) Veröffentlichungstag der Anmeldung:
**26.02.86 Patentblatt 86/09**

(45) Bekanntmachung des Hinweises auf die Patenterteilung:
**24.07.91 Patentblatt 91/30**

(84) Benannte Vertragsstaaten:
**AT BE CH DE FR GB IT LI NL SE**

(56) Entgegenhaltungen:
**EP-A- 0 048 681**
**DE-A- 2 145 090**
**DE-A- 2 853 647**

(73) Patentinhaber: **BAYER AG**
**W-5090 Leverkusen 1 Bayerwerk(DE)**

(72) Erfinder: **Walkowiak, Michael, Dr.**
**Albertus-Magnus-Strasse 10**
**W-5090 Leverkusen(DE)**
Erfinder: **Nehren, Klaus**
**Charlottenburger-Strasse 32**
**W-5090 Leverkusen(DE)**
Erfinder: **Podszun, Wolfgang, Dr.**
**Wolfskaul 4**
**W-5000 Koeln 80(DE)**
Erfinder: **Finger, Werner, Prof. Dr.**
**Kurt-Schumacher-Ring 148**
**W-5090 Leverkusen(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft die Verwendung von nachstehend näher definieren porösen anorganischen Teilchen, insbesondere aus $SiO_2$ oder Silikaten, als Füllstoffe für polymerisierbare Dentalmassen. Die erfindungsgemäßen Massen können z.B. für zahnärztliche Restaurierungs- und Reparaturarbeiten, als Kronen- und Brücken-Materialien sowie zur Herstellung künstlicher Zähne eingesetzt werden.

Härtbare, füllstoffhaltige Dentalmassen auf Basis von ethylenisch ungesättigten, polymerisierbaren Monomeren (insbesondere mono-, di- und auch polyfunktionellen Estern der Acryl- und Methacrylsäure) sind z.B. aus US-PS 3 066 112, US-PS 3 926 906 und GB-PS 1 544 776 bekannt. Die (anorganischen) Füllstoffe müssen den polymerisierbaren Massen zugesetzt werden, um deren polymerisationsschrumpf zu verringern, den thermischen Expansionskoeffizienten zu erniedrigen und die Härte der erhaltenen Polymerisate zu erhöhen.

Der Anteil an inerten anorganischen Füllstoffen kann in derartigen Dentalmaterialien bis zu über 80 % der Gesamtmasse betragen. Als Füllstoffe werden z.B. Quarz, Quarzglas oder Silikatgläser, wie Lithiumaluminiumsilikat oder Bariumsilikatglas als feine Pulver verwendet. Die Korngrößen dieser Füllstoffe liegen im Bereich von 1 bis ca. 100 $\mu$m, wobei im allgemeinen der mittlere Teilchendurchmesser in der Größenordnung von etwa 10 $\mu$m liegt.

Nachteilig an der Verwendung dieser bekannten Füllstoffe ist die noch nicht befriedigende Abriebfestigkeit der daraus hergestellten Dentalmaterialien sowie ihre Oberflächenrauhigkeit beim Einsatz als Zahnfüllungen. Infolge ihres schlechten Abriebverhaltens konnten die Zahnfüllmaterialien mit derartigen Füllstoffen nicht im Seitenzahnbereich eingesetzt werden, so daß dort heute die Amalgamfüllungen noch vorherrschen.

Die Oberflächenrauhigkeit der genannten Materialien führt aber auch bei der Verwendung im Frontzahngebiet zu Problemen, da hier die Ablagerung von Zahnbelag gefördert wird und dadurch sowohl Verfärbungen als auch marginale Sekundärkaries verursacht werden können.

Um die Nachteile der Oberflächenrauhigkeit zu beseitigen, wird in DE-PS 2 403 211 die Verwendung von flammpyrolytisch gewonnenem hochdispersem Siliciumdioxid als Füllstoff vorgeschlagen, dessen Teilchengröße im Bereich von 10-400 nm liegen soll; die BET-Oberfläche soll kleiner als 200 $m^2/g$ sein; ausdrücklich wird auf die Unbrauchbarkeit gefällter Kieselsäuren hingewiesen.

In EP-A 0 060 911 wird eine Kombination von Makro- und Mikrofüller-Composites beschrieben. Gemäß EP-A 0 040 232 erhält man durch Granulierung von pyrogenem oder gefälltem Siliciumdioxid mit Wasserglas oder Borsäure stabile Agglomerate, die höhere Füllgrade erlauben und polierbar sind. Auch die Verwendung mikroporöser Füllstoffe ist an sich bekannt. Poröse Gläser sind z.B. beschrieben in US-PS 2 106 744, US-PS 3 549 524 und US-PS 4 306 913. Durch Sintern von Glasfasern kann man, wie in EP-A 0 048 681 beschrieben, ebenfalls poröse Füllstoffe erhalten. Diese konventionellen mikroporösen Füllstoffe haben eine spezifische Oberfläche von maximal etwa 20 $m^2/g$.

Ein entscheidender Schwachpunkt der bisher bekannten Makrofüller ist der schlechte Verbund zwischen Füllstoffoberfläche und Polymermatrix, der sich durch Oberflächenbehandlung nur partiell verbessern läßt. Zwar läßt sich der Verbund durch Vergrößerung der Oberfläche bzw. der Porenzahl erhöhen, aber auch dann zeigen Bruchbilder im REM ebenso wie bei den Agglomeraten einen unzureichenden Verbund zwischen Fülleroberfläche und Polymermatrix. Eine Zusammenfassung der Vor- und Nachteile der einzelnen Systeme hinsichtlich ihres klinischen Verhaltens ist von Lutz, Phillips, Roulet, Imfeld in Schweiz. Mschr. Zahnheilkunde 93, 914-929 (1983) beschrieben.

Es wurde nun überraschenderweise gefunden, daß an sich bekannte synthetische amorphe hochporöse Teilchen, insbesondere Kieselsäuren, wie sie z.B` in DE-OS 2 145 090 (US-PS 3 959 174), DE-OS 2 124 223 und DE-OS 2 853 647 als Mattierungsmittel beschrieben sind, hervorragende Eigenschaften als Füllstoffe zeigen.

Gegenstand der Erfindung ist die Verwendung mikroporöser anorganischer Füllstoffe in polymerisierbaren Dentalmassen, dadurch gekennzeichnet, daß die Füllstoffe

a) eine mittlere Teilchengröße von 0,5 - 50 $\mu$, vorzugsweise 1-20 $\mu$;
b) eine BET-Oberfläche von mindestens 200 $m^2/g$, vorzugsweise 300-600 $m^2/g$;
c) ein Porenvolumen von 0,7-5 ml/g, vorzugsweise 1-3 ml/g; und
d) einen Porendurchmesser von 10-50 nm, vorzugsweise ca. 20 nm,

aufweisen.

Gegenstand der Erfindung sind weiterhin polymerisierbare Dentalmassen, enthaltend 20 bis 65 Gew.-%, vorzugsweise 30 bis 60 Gew.-% eines auf diesem Gebiet üblichen polymerisierbaren Monomeren und 10 bis 60 Gew.-%, vorzugsweise 30 bis 50 Gew.-%, eines anorganischen Füllstoffes, sowie gegebenenfalls auf diesem Gebiet übliche Zuschlagstoffe, dadurch gekennzeichnet, daß der Füllstoff die oben angegebenen Kriterien erfüllt.

Gegenstand der Erfindung ist schließlich auch ein Verfahren zur Herstellung von Dental-Formkörpern, dadurch gekennzeichnet, daß man eine erfindungsgemäße Masse unter Formgebung polymerisiert.

Obwohl die erfindungsgemäß zu verwendenden mikroporösen Füllstoffe einen relativ großen mittleren Teilchendurchmesser aufweisen, sind die erfindungsgemäß hergestellten Formkörper (insbesondere Zahnfüllungen) hervorragend polierbar. Aufnahmen von Bruchflächen im REM zeigen eine so glatte Oberfläche, wie man sie sonst nur von homogen aufgebauten Materialien kennt.

Überraschenderweise sind die erfindungsgemäß hergestellten Formkörper auch weitgehend transparent, obwohl die Brechungsindices z.B. von amorpher Kieselsäure (1,46) und gängigen, polymerisierten Methacrylsäureestern wie Bis-GMA/Triethylenglykoldimethacrylat (1,55) merklich verschieden sind. Entgegen dem in DE-OS 2 403 211 geäußerten Vorurteil zeigen gerade solche Füllstoffe die besten Eigenschaften, deren BET-Oberfläche größer als 200 $m^2/g$ (bevorzugt 300-600 $m^2/g$) ist.

Die erfindungsgemäß zu verwendenden mikroporösen Füllstoffe sind im Handel erhältlich (z.B. Syloid $^R$ -Typen der Firma W.R. Grace & Co., New York). Sie können z.B. nach den in DE-OS 2 145 090 und DE-OS 2 853 647 angegebenen Verfahren hergestellt werden. Als Ausgangsmaterial kommen hierfür im Prinzip alle gelbildenden anorganischen Oxide und Salze in Betracht, insbesondere $SiO_2$, $Al_2O_3$ und Silikate (bevorzugt Ca-Silikate).

Die Füllstoffe können gegebenenfalls vor ihrer erfindungsgemäßen Verwendung in an sich bekannter Weise oberflächenbehandelt werden. Vorzugsweise werden zu diesem Zweck Organosiliciumverbindungen in einer Menge von 5-40 Gew.-%, bezogen auf Füllstoff, eingesetzt. Geeignete Verbindungen sind z.B. Vinyltriethoxysilan, Vinyltrichlorsilan, Vinyltrimethoxysilan, Allyldimethylchlorsilan, $\gamma$-Methacryloxypropyltrimethoxysilan, $\beta$-(3,4-Epoxycyclohexyl-ethyltrimethoxysilan, $\gamma$-Glycidoxypropyltrimethoxysilan, aber auch Disilazane wie Hexamethyldisilazan oder Vinyldisilazane.

Die erfindungsgemäß zu verwendenden Füllstoffe können alleine oder auch in Verbindung mit anderen an sich bekannten, vorzugsweise mikrofeinen (Teilchengröße <500 nm), Füllstoffen, die gegebenenfalls ebenfalls silanisiert oder mit (Meth-)acrylaten gepfropft sein können, verwendet werden. Diese Füllstoffe können z.B. in Mengen von 1-40 Gew.-%, vorzugsweise 5-20 Gew.-%, bezogen auf polymerisierbare Masse, zugesetzt werden.

Die in den erfindungsgemäßen Massen zu verwendenden Monomeren weisen mindestens eine radikalisch polymerisierbare Doppelbindung auf. Vorzugsweise werden Monomere mit mehr als einer Doppelbindung und Siedepunkten über 100 °C bei 13 mbar, allein oder gegebenenfalls im Gemisch mit monofunktionellen Monomeren verwendet. Dadurch werden hochvernetzte Polymerisate oder Copolymerisate erhalten. Die Molgewichte der Monomeren können zwischen etwa 70 und 20000 liegen, vorzugsweise zwischen etwa 150 und 1000. Die Viskosität der Monomeren kann durch geeignete Abmischung von höherviskosen bzw. höhermolekularen Monomeren mit niedrigviskosen Monomeren eingestellt werden. Die Monomeren enthalten gegebenenfalls geringe Mengen an Polymerisationsinhibitoren, wie z.B. 0,01 - 0,2 % 2,6-Di-t-butyl-p-kresol.

Als erfindungsgemäß zu verwendende polymerisierbare Monomere kommen beispielsweise in Frage:

Ester von ungesättigten Mono- oder Dicarbonsäuren, z.B. Ester der Acryl-, Methacryl-, $\alpha$-Cyanacryl-, Croton-, Zimt-, Sorbin-, Malein-, Fumar- oder Itaconsäure mit aliphatischen, cycloaliphatischen oder aromatischaliphatischen ein- bis vierwertigen Alkoholen mit 2-30 Kohlenstoffatomen, z.B. Methyl (meth-)acrylat, n-, i- und t-Butyl (meth-)acrylat, 2-Ethylhexyl (meth)acrylat, Lauryl(meth)acrylat, Dihydrodicyclopentadienyl-(meth-)acrylat, Dihydroxymethyl-tricyclo [5,2,1,0,2,6] decandi(meth)acrylat gemäß der DE-PS 2 200 021, Methylglykoldi(meth)acrylat, Hydroxyethyl(meth)acrylat, Hydroxypropyl(meth)acrylat, Ethylenglykoldi(meth)-acrylat, Diethylenglykoldi(meth)acrylat, Triethylenglykoldi(meth)acrylat, Neopentylglykoldi(meth)acrylat, 1,4-Dimethylolcyclohexandi(meth)acrylat, Pentaerythrit-tri- und tetra(meth)acrylat, Trimethylolpropan tri(meth)-acrylat, Ethyl-$\alpha$-cyanoacrylat, Ethylcrotonat, Ethylsorbinat, Diethylmaleinat, Diethylfumarat sowie das Di-(meth)acrylat des oxalkylierten Bisphenol A gemäß US-PSS 3 810 938 und 3 923 740, Di(meth)-acrylsäureester von oxalkyliertem Trimethylolpropan oder Pentaerythrit gemäß US-PS 3 380 831 und auch die (Meth)acrylester von oxyalkylierten Di-(hydroxymethyl)-tricyclo [5,2,1,0,2,6]-decanen, wie sie in DE-OS 2 931 925 und DE-OS 2 931 926 beschrieben werden.

Weitere in den erfindungsgemäßen Massen einsetzbare Monomere sind Amide der (Meth)acrylsäure, die gegebenenfalls am Stickstoffatom mit Alkyl-, Alkoxyalkyl- oder Hydroxyalkylresten substituiert sein können, wie z.B. N-Isobutylacrylamid, Diacetonacrylamid, N-Methylolacrylamid, N-Methoxymethylacrylamid, N-Butoxymethylmethacrylamid, Ethylenglykolbis-(N-methylolacrylamid)ether und Methylen-bis-acrylamid; Triacrylformal; Vinylester von Mono- und Dicarbonsäuren mit 2 bis 20 Kohlenstoffatomen, z.B. Vinylacetat, Vinylpropionat, 2-Ethylhexansäure-vinylester, Versaticsäurevinylester und Divinyladipat; Vinylether von ein- oder zweiwertigen Alkoholen mit 3 bis 20 Kohlenstoffatomen, z.B. Isobutylvinylether, Octadecylvinylether, Ethylenglykoldivinylether und Diethylenglykoldivinylether; Mono-N-Vinylverbindungen, z.B. N-Vinylpyrrolidon, N-Vinylpiperidon, N-Vinylcaprolactam, N-Vinylmorpholin, N-Vinyloxazolidon, N-Vinylsuccinimid, N-Methyl-N-vinylformamid und N-Vinylcarbazol; Allylether und -ester, z.B. Trimethylolpropandiallylether, Trimethylolproptriallylether, Allyl(meth-)acrylat, Diallylmaleinat, Diallylphthalat und dessen Präpolymere sowie beliebige Gemische aller aufgeführten ungesättigten Verbindungen.

Für medizinische Zwecke sind die Epoxidacrylate und Urethanacrylate besonders geeignet. Als Beispiele für solche Verbindungen seien aufgeführt:

a) Reaktionsprodukte aus monofunktionellen Epoxiden und (Meth)acrylsäure gemäß US-PS 2 484 487 und US-PS 2 575 440;

b) Reaktionsprodukte aus bifunkionellen Epoxiden und ungesättigten Fettsäuren nach US-PS 2 456 408;

c) Reaktionsprodukte aus polyfunktionellen aromatischen oder aliphatischen Glycidethern und (Meth-) acrylsäure gemäß US-PSS 3 179 623, 3 066 112, 2 824 851 und DE-PS 1 644 817;

d) Reaktionsprodukte aus Epoxidharzen und (Meth)acrylsäurechlorid gemäß US-PS 3 427 161 und US-PS 2 890 202;

e) ungesättigte Polyurethane (Urethanacrylate) und Polyharnstoffe aus Hydroxyalkyl(meth)acrylaten, Aminoalkyl(meth)acrylaten und gegebenenfalls Polyolen oder Polyaminen, wie sie in den US-PSS 3 425 988, 3 709 866, 3 629 187, 4 089 763 und 4 110 184 sowie den DE-PSS 1 644 798, 1 644 797, DOS 2 357 402, 2 357 324, 2 358 948 beschrieben sind.

Weitere Beispiele geeigneter Comonomere sind der nachstehenden Zusammenstellung zu entnehmen; in den Strukturformeln stehen

R für $CH_2=C(CH_3)-CO-$ oder $CH_2=CH-CO-$

R' für H oder $CH_2$-OR
n für eine Zahl zwischen 1 und 4 und
m für eine Zahl zwischen 0 und 4.

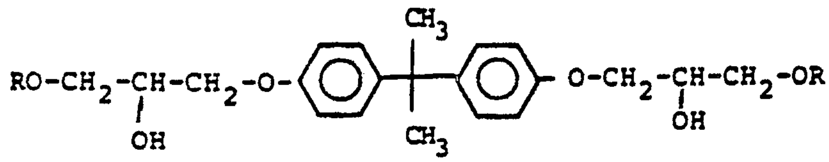

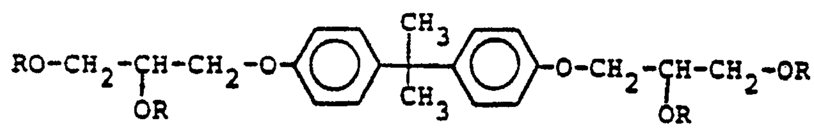

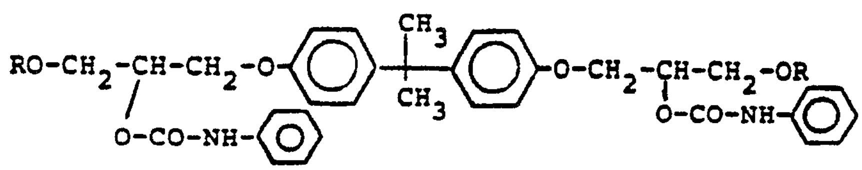

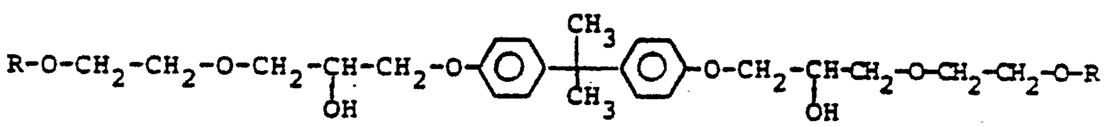

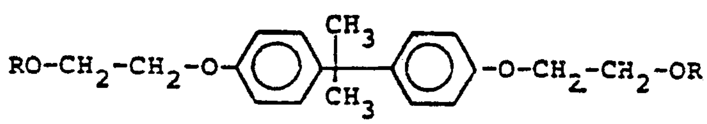

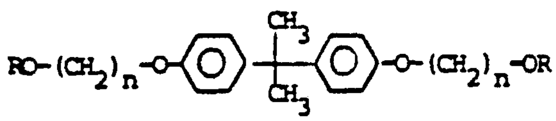

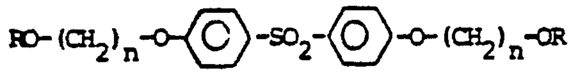

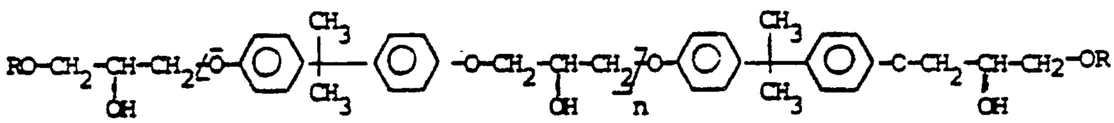

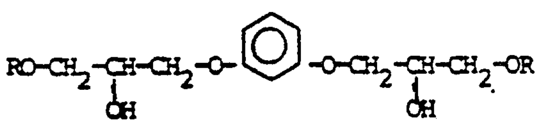

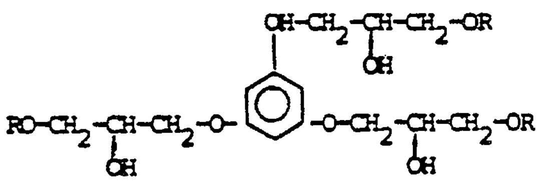

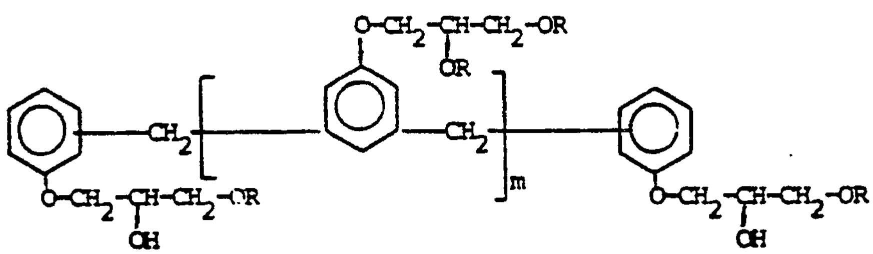

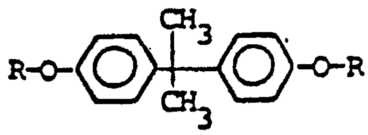

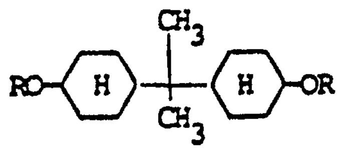

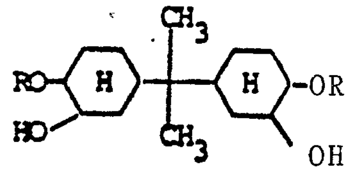

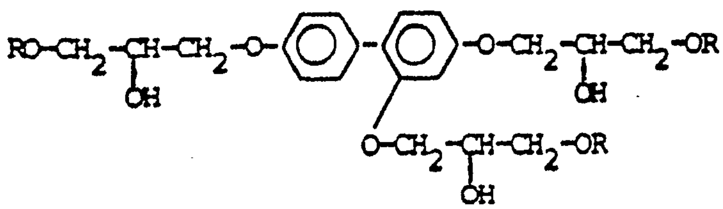

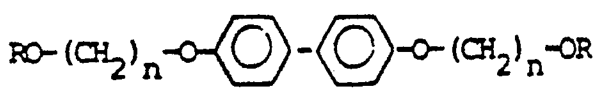

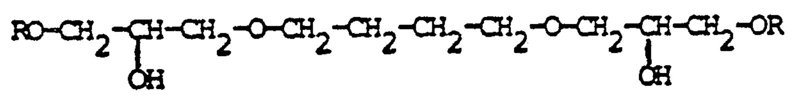

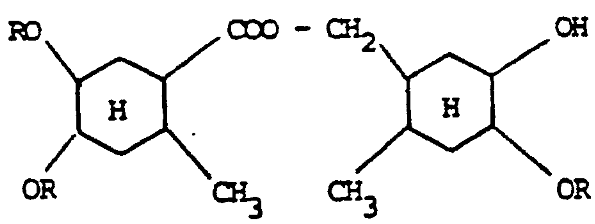

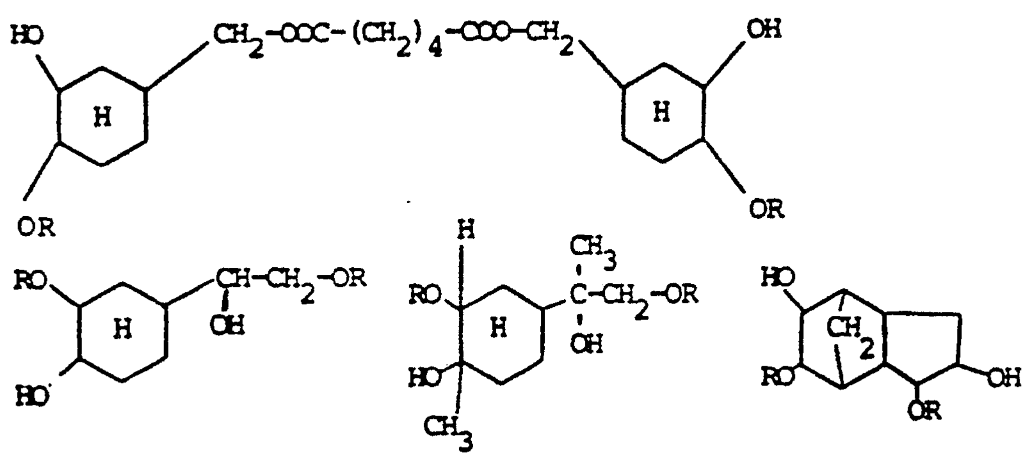

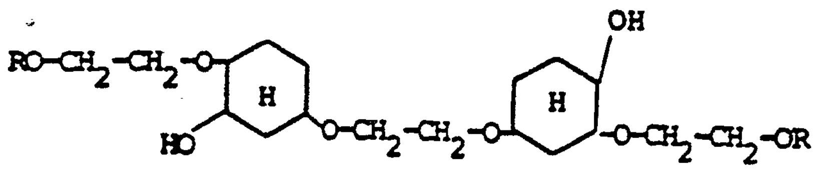

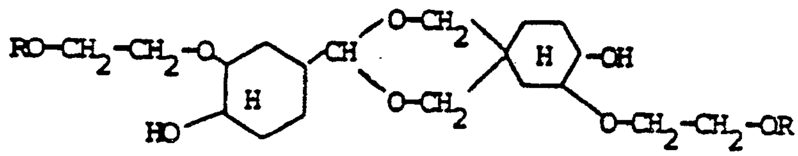

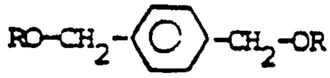

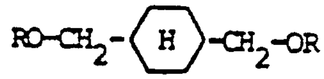

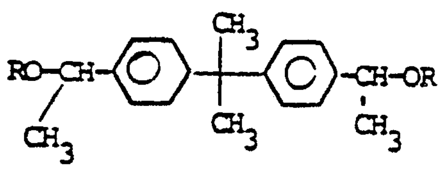

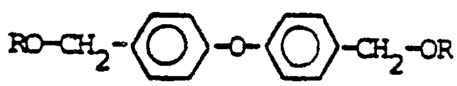

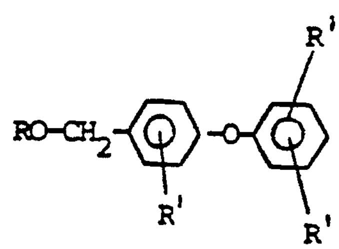

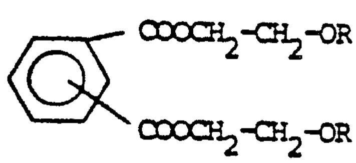

in der ortho-, meta- oder para-Form

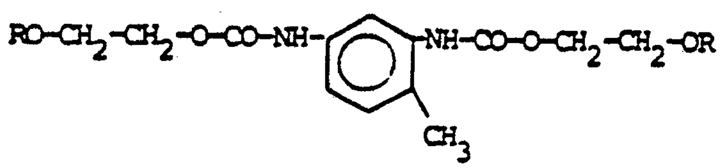

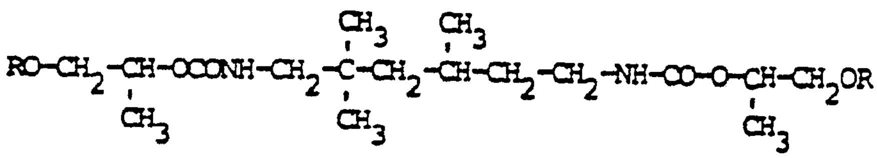

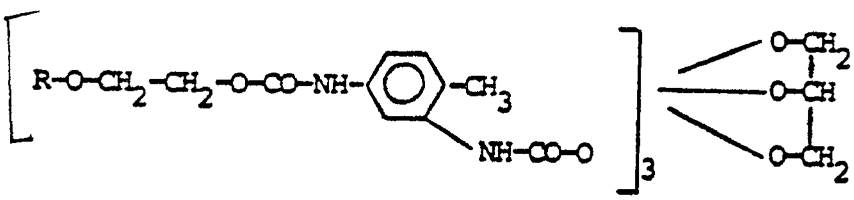

sowie Verbindungen der allgemeinen Formel

$R\text{-}(O\text{-}D\text{-}O\text{-}X)_n\text{-}OD\text{-}OR$

wobei HO-D-OH ein Polyol und HO-X-OH eine Dicarbonsäure darstellen, die jeweils gesättigt oder ungesättigt, cyclisch oder acyclisch sein können.

Je nach Anwendungszweck können in den erfindungsgemäßen Massen noch andere Stoffe mitverwendet werden, wie z.B. weitere anorganische und/oder organische Füllstoffe und Pigmente, Stabilisatoren, Farbstoffe, spezielle Lichtschutzmittel, Fluroeszenzmittel, Weichmacher sowie lösliche, quellbare oder unlösliche hochmolekulare Verbindungen.

Als Dental-Werkstoff sehr gut geeignete Pasten erhält man, wenn als Ester dar Methacrylsäure zumindest in Anteilen Verbindungen vom Typ des Bis-GMA der Formel

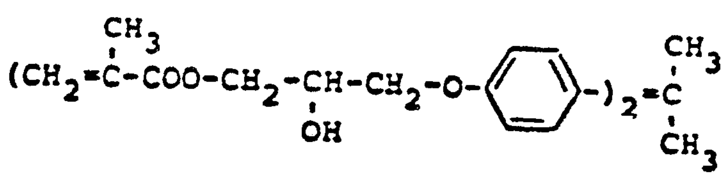

eingesetzt werden.

Zahnfüllmassen mit guter Konsistenz und einem hohen Niveau der mechanischen Festigkeit werden besonders dann erhalten, wenn man als Ester der Methacrylsäure Mischungen aus verschiedenen Methacrylsäureestern verwendet, z.B. Mischungen von 20-70 Gew.-Teilen Bis-GMA und 30-80 Gew.-Teilen Triethylenglykoldimethacrylat (TEGDMA).

Als Starterzusätze für die Einleitung der Polymerisation können die üblichen Startersysteme verwendet werden, d.h. Radikale liefernde Systeme, die eine radikalische Polymerisation auslösen können. Radikale liefernde Systeme sind besonders Peroxide oder aliphatische Azoverbindungen, beispielsweise Benzoylperoxid, Laurylperoxid oder Azoisobuttersäuredinitril, die üblicherweise in Mengen von 0,1 bis 5 Gew.-% eingesetzt werden. Während die Härtung bei erhöhter Temperatur allein durch Peroxide oder andere Radikalstarter durchgeführt werden kann, ist zur Härtung bei Raumtemperatur im allgemeinen ein Zusatz von Beschleunigern, vorzugsweise aromatischen Aminen, notwendig. Geeignete Beschleuniger sind z.B.

N,N-substituierte Toluidine und Xylidine, wie N,N-Dimethyl-p-toluidin oder N,N-Bis(2-hydroxy-ethyl)xylidin. Gute Aushärtungszeiten erzielt man mit 0,5 - 3 % Aminzusatz. Eine günstige Darbietungsform für ein mit Peroxid und Beschleuniger aktiviertes System ist die 2-Pasten-Form, wobei eine Paste den Radikalstarter und die andere den Beschleuniger enthält und die Aushärtung durch Mischen beider Pasten eingeleitet wird.

Es ist jedoch auch möglich, einphasige Präparate herzustellen, die unter Einwirkung von Licht, beispielsweise UV, sichtbares Licht oder Laserlicht, polymerisieren und dann selbstverständlich einen Photopolymerisationsinitiator und gegebenenfalls auch einen Beschleuniger dafür enthalten.

Entsprechende Photopolymerisationsinitiatoren sind bekannt, vorzugsweise handelt es sich dabei um Carbonylverbindungen, wie Benzoin und dessen Derivate, insbesondere Benzoinmethylether, Benzil und Benzilderivate, beispielsweise 4,4-Oxidibenzil oder andere Dicarbonylverbindungen, z.B. Diacetyl, 2,3-Pentandion oder Metallcarbonyle, Chinone oder deren Derivate. Der Anteil an solchen Photopolymerisationsinitiatoren beträgt vorzugsweise etwa 0,01 bis etwa 5 Gew.-% der Gesamtzusammensetzung.

Diese mit Licht härtbaren, d.h. photopolymerisierbaren Präparate enthalten vorzugsweise auch noch Substanzen, die in Gegenwart von Photopolymerisationsinitiatoren die Polymerisationsreaktion beschleunigen. Bekannte Beschleuniger sind beispielsweise aromatische Amine wie p-Toluidin und Dimethyl-p-toluidin, Trialkylamine wie Trihexylamin, Polyamine, wie N,N,N',N'-Tetraalkylalkylendiamine, Barbitursäure und Dialkylbarbitursäuren und Sulfimide, vorzugsweise in einer Menge von etwa 0,01 bis etwa 5 Gew.-% der Gesamtzusammensetzung.

Es ist schließlich zweckmäßig, dentalen Füllungsmaterialien auf Kunststoffbasis UV-Stabilisatoren zuzusetzen, um das Nachdunkeln während des Alterns der Füllungen zu vermeiden.

Ein besonders geeigneter UV-Stabilisator ist 2-Hydroxy-4-methoxybenzophenon. Ein weiteres bevorzugtes Material ist 2-(2'-Hydroxy-5'-methylphenyl)-benzotriazol; jedoch ist prinzipiell jedes physiologisch inerte UV-absorbierende Agens für diesen Zweck geeignet. So seien beispielhaft noch Hydrochinon, p-Benzochinon, p-Butylhydroxytoluol u.ä. genannt. Die letztere Verbindung kann beispielsweise auch als Antioxidans in der Füllung wirken.

Eine Übersicht über die in dentalen Füllungsmaterialien üblicherweise zum Einsatz gelangenden Substanzen findet sich in dem Artikel von R.L. Bowen im Journal of Dental Research, Vol. 58/5 (Mai 1979), S. 1493 bis 1503, sowie der daran angeschlossenen Ergänzung von J.F. Lann, S. 1504 bis 1506, sowie den dort zitierten Literaturstellen.

Zur Einstellung eines möglichst naturgetreuen Eindrucks der gefüllten Zahnflächen enthalten Composite-Materialien erforderlichenfalls auch einen geringen Anteil an Farbstoffen oder Pigmenten.

Bei Anwendung großer Anteile von mehrfunktionellen Monomeren (vernetzern) und für bestimmte Einsatzzwecke kann es vorteilhaft sein, den erfindungsgemäßen polymerisierbaren Massen Weichmacher zur Verringerung der Sprödigkeit zuzusetzen. Gut geeignet sind in erster Linie an sich bekannte hochmolekulare Weichmacher, besonders solche auf Basis von Polyurethanen, Polycarbonaten, Polyestern und Polyethern. Bevorzugt werden Polyester und Polyestercarbonate, die in DE-OS 3 316 851 beschrieben werden.

Die erfindungsgemäßen Massen werden insbesondere im Dentalbereich für die Herstellung von mit dem menschlichen oder tierischen Körper in kontakt kommenden Polymerisaten eingesetzt, z.B. als Knochenzemente, Dentalwerkstoffe und medizinische Versiegelungsmassen. Im Zusammenhang mit Dentalwerkstoffen seien vor allem Zahnrestaurationsmassen (insbesondere auch Zahn-Füllungsmassen), Kronen, Brücken, Verblendungen und ähnliche Zahnersatzteile und auch künstliche Zähne genannt.

Herstellung der erfindungsgemäß zu verwendenden hochporösen Füllstoffe:

1072 g Natriumsilikatlösung,
172 g Ammoniumhydroxidlösung (29 %ig) und
1756 g Wasser

wurden vermischt, so daß insgesamt 3000 g Lösung erhalten wurden. Die Lösung enthielt 300 g Kieselsäure und 50 g Ammoniak, was einer Siliciumdioxidkonzentration von 10 % und einem $SiO_2$ : $NH_2$ - Verhältnis von 6 entspricht. Kohlendioxid wurde in diese Lösung eingeleitet, wodurch der pH-Wert auf 10,91 fiel. Die Lösung gelierte innerhalb von 4-6 Minuten. Dieses Kieselsäurehydrogel wurde 10 min lang ohne Rühren und 50 min lang unter Rühren gealtert. Kohlendioxid wurde während etwa einer Stunde zugeführt, bis der pH-Wert 9,0 erreichte.

Anschließend wurde Schwefelsäure zugesetzt, um das in der Natriumsilikatlösung gebildete Natriumcarbonat zu neutralisieren.

Die Lösung wurde anschließend 16 Stunden bei 60 °C gealtert. Das Produkt wurde getrocknet und wies die folgenden Eigenschaften auf:

| | | |
|---|---|---|
| Gesamtgehalt an flüchtigen Bestandteilen bei 954°C | 7 | % |
| $Na_2O$ (Trockenbasis) | 0,03 | % |
| $SO_4$ (Trockenbasis) | 0,02 | % |
| Oberfläche (BET) | 400 | $m^2/g$ |
| Porenvolumen | 2,4 | $cm^3/g$ |
| Porendurchmesser | ca. 20 | nm |

Die getrocknete Kieselsäure wurde in eine 10 cm Jet Pulverizer-Strahlmühle unter Verwendung von Luft mit einer Einlaßtemperatur von 427 °C und einem Druck von 9,84 atü eingespeist. Der Injektionsdruck für die Kieselsäure lag bei 10,5 atü. Die abfließende Kieselsäure wurde in einem Sackabscheider aufgefangen. Die gebildete Kieselsäure wies eine durchschnittliche Teilchengröße von 7 µm auf.

Durch Variation der Silikatkonzentration und des Silikat/ Ammoniak-Verhältnisses läßt sich, wie in DE-OS 2 145 090 beschrieben, das Porenvolumen des Füllstoffs gezielt einstellen. Die Teilchengröße kann durch geeignete Änderung der Mahlbedingungen variiert werden.

Beispiel 1

Es wird eine Lösung hergestellt aus:

| | | |
|---|---|---|
| 31,0 | g | Bis-GMA |
| 19,0 | g | TEGDMA |
| 0,25 | g | N,N-Dialkyl-p-dimethylaminobenzolsulfonsäureamid |
| 0,1 | g | Campherchinon |
| 0,06 | g | Benzildimethylketal und |
| 0,05 | g | Jonol |

In 40 g obiger Lösung werden 26,0 g einer mit 15,5 Gew.-% γ-Methacryloxypropyltrimethoxysilan silanisierten Kieselsäure mit folgenden Kennzahlen gegeben:

| | | |
|---|---|---|
| mittlere Teilchengröße: | 5 | µ |
| BET-Oberfläche: | 400 | $m^2/g$ |
| Porenvolumen: | 1,8 | ml/g |
| Porendurchmesser: | ca. 20 | nm |

und zu einer homogenen Masse geknetet.

Nach Härtung der Masse mit sichtbarem Licht erhält man hoch transparente Probekörper mit einer Biegefestigkeit (nach DIN 13 922) von 83,06 $N/mm^2$, mit einem Biegemodul von 3310 $N/mm^2$ und einer diametralen Zugfestigkeit (nach ADA 27) von 35,6 $N/mm^2$.

Beispiel 2

Im Vakuum werden zu

| | | |
|---|---|---|
| 80 | g | der Kieselsäure aus Beispiel 1 und |
| 16,8 | g | einer pyrogenen Kieselsäure mit einer BET-Oberfläche von 50 $m^2/g$ und einer Primärteilchengröße von 45 nm |
| 39,2 | g | TEGDMA zugegeben. |

| | | | |
|---|---|---|---|
| Zu | 34,6 | g | dieses Gemisches werden |
| | 20,8 | g | Bis-GMA |
| | 0,15 | g | Sulfonamid aus Beispiel 1 |
| | 0,06 | g | Campherchinon und |
| | 0,04 | g | Benzildimethylketal |

zugegeben und zu einer Paste verarbeitet.

Nach Härtung der Paste mit sichtbarem Licht erhält man transparente Probekörper mit folgenden mechanischen Eigenschaften:

| | | |
|---|---|---|
| Biegefestigkeit: | 81,4 | $N/mm^2$ |
| Biegemodul: | 3738 | $N/mm^2$ |
| Diametrale Zugfestigkeit: | 33 | $N/mm^2$ |

Beispiel 3 (Vergleich)

Es wird eine Lösung hergestellt aus:

| | | |
|---|---|---|
| 67,2 | g | Bis-GMA |
| 41,2 | g | TEGDMA |
| 1,1 | g | Tinuvin P |
| 0,04 | g | Jonol |
| 0,54 | g | Sulfonamid aus Beispiel 1 |
| 0,14 | g | Benzildimethylketal und |
| 0,22 | g | Campherchinon |

Zu 62,0 g obiger Lösung werden 85,0 g einer mit 9,3 Gew.-% γ-Methacryloxyropyltrimethoxysilan silanisierten Kieselsäure (Syloid® Al 1 von Grace) mit folgenden Kenndaten gegeben:

| | | |
|---|---|---|
| Porenvolumen: | 0,4 | ml/g |
| mittlere Teilchengröße: | 8 | μ |
| BET-Oberfläche: | 750 | $m^2/g$ |
| Porendurchmesser: | ~ 4 | nm |

Man erhält eine opake Paste.
Polymerisationstiefe (Translux-Lampe) nach 60 sec.

| | | |
|---|---|---|
| Belichtung: | 5,0 | mm |
| Biegefestigkeit: | 33,7 | $N/mm^2$ |
| Biegemodul: | 4186 | $N/mm^2$ |
| Diametrale Zugfestigkeit: | 22,9 | $N/mm^2$ |

Die Paste ist gut polierbar, aber wegen der großen Opazität nicht als Zahnfüllmaterial brauchbar.

Beispiel 4

100 g der aktivierten Lösung aus Beispiel 3 werden mit 51 g einer mit 12 Gew.-% γ-Methacryloxypropyltrimethoxysilan silanisierten Kieselsäure mit folgenden Kenndaten zu einer Paste verarbeitet:

| | | |
|---|---|---|
| Porenvolumen: | 1,6 | ml/g |
| mittlere Teilchendurchmesser: | 2 | μ |
| BET-Oberfläche: | 400 | $m^2/g$ |
| Porendurchmesser: | ca. 18 | nm |

Man erhält nach der Härtung mit Licht transparente, sehr gut polierbare Probekörper.

| | | |
|---|---|---|
| Polymerisationstiefe (Translux) nach 30 sec.: | 12 | mm |
| Biegefestigkeit: | 76,6 | $N/mm^2$ |
| Biegemodul: | 3697 | $N/mm^2$ |
| Diametrale Zugfestigkeit: | 37,7 | $N/mm^2$ |

Beispiel 5

120 g der aktivierten Lösung aus Beispiel 3 werden mit 66 g einer mit 18 Gew.-% γ-Methacryloxypropyltrimethoxysilan silanisierten Kieselsäure mit folgenden Kenndaten zu einer Paste verarbeitet:

| | | |
|---|---|---|
| Porenvolumen: | 1,2 | ml/g |
| mittlere Teilchengröße: | 12 | μ |
| BET-Oberfläche: | 400 | $m^2/g$ |
| Porendurchmesser: | ca. 13 | nm |

Man erhält nach der Härtung mit Licht ausreichend transparente, sehr gut polierbare Probekörper.

| | | |
|---|---|---|
| Polymerisationstiefe (Translux) nach 30 sec.: | 8,8 | mm |
| 60 sec.: | 11 | mm |
| Biegefestigkeit: | 69,3 | $N/mm^2$ |
| Biegemodul | 4020 | $N/mm^2$ |
| Diametrale Zugfestigkeit: | 37 | $N/mm^2$ |

Beispiel 6

A) Zu einer Mischung aus

| | | |
|---|---|---|
| 5,5 | g | Bis-GMA |
| 2,3 | g | TEGDMA und |
| 2,0 | g | Trimethylolpropantrimethacrylat |

werden

| | | |
|---|---|---|
| 0,2 | g | Benzoylperoxid und |
| 5,4 | g | einer mit Hexamethyldisilazan behandelten Kieselsäure (10,5 Gew.-% Trimethylsilylgruppen enthaltend) gegeben: |

| | |
|---|---|
| Porendurchmesser: | ca. 20 nm |
| Porenvolumen: | 1,8 ml/g |
| mittlerer Teilchendurchmesser | 5 µ |
| BET-Oberfläche: | 500 $m^2/g$ |

und zu einer Paste verarbeitet. B) Zu

| | | |
|---|---|---|
| 9,91 | g | Monomergemisch der gleichen Zusammensetzung wie unter A) werden |
| 0,09 | g | N-Methyl-N-ß-(methylcarbamoyloxypropyl-)3,5-dimethylanilin und |
| 5,5 | g | der unter A) beschriebenen Kieselsäure zugegeben und zu einer zweiten Paste verarbeitet. |

Durch Mischen der Pasten A) und B) im Verhältnis 1:1 erhält man Probekörper mit folgenden physikalischen Eigenschaften:

| | |
|---|---|
| Biegefestigkeit: | 58,2 N/mm² |
| Biegemodul: | 3450 N/mm² |
| diametrale Zugfestigkeit | 30,5 N/mm² |

Beispiel 7

A) Zu einer Monomermischung aus

5,3 g Bis-GMA
3,5 g TEGDMA und
1,0 g Pentaerythritol-tetramethacrylat werden
0,2 g Benzoylperoxid sowie
4,5 g der im Beispiel 6 A) beschriebenen Kieselsäure gegeben und zu einer Paste verarbeitet.

B) Zu

9,9 g Monomergemisch wie in A) werden
0,7 g N,N-dimethyltoluidin sowie
4,6 g der Kieselsäure aus Beispiel 6 A) gegeben und zu einer zweiten Paste verarbeitet.

Durch Mischen der Pasten A) und B) im Verhältnis 1:1 erhält man Probekörper mit folgenden physikalischen Eigenschaften:

| | |
|---|---|
| Biegefestigkeit: | 52,8 N/mm² |
| Biegemodul: | 3517 N/mm² |
| diametrale Zugfestigkeit | 32,3 N/mm² |

Beispiel 8

A) Aus

9,8 g Methacrylester des oxalkylierten Bis-hydroxy-methyl-tricyclo [5.2.1.0$^{2.6}$]decans (entsprechend EP-A-0 023 685, Beispiel 1)
0,2 g Benzoylperoxid und
4,8 g der im Beispiel 4 beschriebenen Kieselsäure wird eine Paste hergestellt.

B) Aus

| | | |
|---|---|---|
| 9,1 | g | des obigen Monomers, |
| 0,9 | g | Bis-(ß-hydroxyethyl)-xylidin und |
| 4,9 | g | der im Beispiel 4 beschriebenen Kieselsäure |

wird eine zweite Paste hergestellt.

Nach Mischen der Pasten A) und B) im Verhältnis 1:1 erhält man Probekörper mit folgenden physikalischen Eigenschaften:

| | |
|---|---|
| Biegefestigkeit: | 62,6 N/mm² |
| Biegemodul: | 3345 N/mm² |
| diametrale Zugfestigkeit | 38,2 N/mm² |

<u>Beispiel 9</u>

A) Aus

| | | |
|---|---|---|
| 6,9 | g | Bis-GMA |
| 2,9 | g | TEGDMA |
| 0,2 | g | Benzoylperoxid und |
| 5,0 | g | der im Beispiel 4 beschriebenen Kieselsäure |

wird eine Paste hergestellt.

B) Aus

| | | |
|---|---|---|
| 6,9 | g | Bis-GMA |
| 2,9 | g | TEGDMA |
| 0,09 | g | N-Methyl-N-ß-(methylcarbamoyloxypropyl-) 3,5-dimethylanilin und |
| 5,0 | g | der im Beispiel 4 beschriebenen Kieselsäure |

wird eine zweite Paste hergestellt.

Nach Mischen der Pasten A) und B) im Verhältnis 1:1 erhält man Probekörper mit folgenden physikalischen Eigenschaften:

| | | |
|---|---|---|
| Biegefestigkeit: | 95,9 | $N/mm^2$ |
| Biegemodul: | 3364 | $N/mm^2$ |
| diametrale Zugfestigkeit: | 37,1 | $N/mm^2$ |

## Patentansprüche

1. Polymerisierbare Dentalmassen, enthaltend 20 bis 65 Gew.-% an auf diesem Gebiet üblichen polymerisierbaren Monomeren mit mindestens einer radikalisch polymerisierbaren Doppelbinding und 10 bis 60 Gew.-% eines mikroporösen Füllstoffs aus $SiO_2$, $Al_2O_3$ oder Calciumsilikat, sowie gegebenenfalls an sich für Dentalmassen bekannte Zuschlagstoffe, dadurch gekennzeichnet, daß der Füllstoff
   a) eine mittlere Teilchengröße von 0,5 - 50 µm,
   b) eine BET-Oberfläche von mindestens 200 $m^2/g$,
   c) ein Porenvolumen von 0,7 - 5 ml/g und
   d) einen Porendurchmesser von 10 - 50 nm
   aufweist.

2. Polymerisierbare Dentalmassen nach Anspruch 1, dadurch gekennzeichnet, daß der Füllstoff silanisiert ist.

3. Polymerisierbare Dentalmassen nach Ansprüchen 1 und 2, dadurch gekennzeichnet, daß das polymerisierbare Monomer ein Gemisch aus mono- und polyfunktionellen Methacrylsäurederivaten ist.

4. Polymerisierbare Dentalmassen nach Ansprüchen 1 bis 3, dadurch gekennzeichnet, daß das polymerisierbare Monomer Bis-GMA enthält.

5. Verwendung von mikroporösem $SiO_2$, $Al_2O_3$ oder Calciumsilikat als Füllstoffe in polymerisierbaren Dentalmassen, die 20 - 65 Gew.-% eines auf diesem Gebiet üblichen polymerisierbaren Monomeren mit mindestens einer radikalisch polymerisierbaren Doppelbindung enthalten, dadurch gekennzeichnet, daß die Füllstoffe
   a) eine mittlere Teilchengröße von 0,5 - 50 µm,
   b) eine BET-Oberfläche von mindestens 200 $m^2/g$,
   c) ein Porenvolumen von 0,7 - 5 ml/g und
   d) einen Porendurchmesser von 10 - 50 nm
   aufweisen.

6. Verwendung nach Anspruch 5 in polymerisierbaren Dental-Werkstoffen zur Durchführung zahntechnischer Restaurierungs- und Reparaturarbeiten.

7. Verwendung nach Anspruch 6 in Zahnfüllungsmassen, Dental-Werkstoffen zur Herstellung künstlicher Zähne oder in Kronen- und Brückenmaterialien.

## Claims

1. Polymerisable dental compositions containing 20 to 65% by weight of polymerisable monomers customary in this field and having at least one double bond which can be subjected to free-radical polymerisation, and 10 to 60% by weight of a microporous filler composed of $SiO_2$, $Al_2O_3$ or calcium silicate, and, if appropriate, additives which are known per se for dental compositions, characterised in that the filler has
   a) an average particle size of 0.5-50 µm;
   b) a BET surface area of at least 200 $m^2/g$;
   c) a pore volume of 0.7-5 ml/g and
   d) a pore diameter of 10-50 nm.

2. Polymerisable dental compositions according to Claim 1, characterised in that the filler is silanised.

3. Polymerisable dental compositions according to Claims 1 and 2, characterised in that the polymerisable monomer is a mixture of monofunctional and polyfunctional methacrylic acid derivatives.

4. Polymerisable dental compositions according to Claims 1 to 3, characterised in that the polymerisable monomer contains bis-GMA.

5. Use of microporous $SiO_2$, $Al_2O_3$ or calcium silicate as fillers in polymerisable dental compositions which contain 20 to 65% by weight of a polymerisable monomer customary in this field and having at least one double bond which can be subjected to free-radical polymerisation, characterised in that the fillers have
   a) an average particle size of 0.5-50 $\mu$m;
   b) a BET surface area of at least 200 $m^2/g$;
   c) a pore volume of 0.7-5 ml/g and
   d) a pore diameter of 10-50 nm.

6. Use according to Claim 5 in polymerisable dental materials for carrying out dental restoration and repair work.

7. Use according to Claim 6 in dental filling materials, dental materials for the production of false teeth or in crown and bridge materials.

**Revendications**

1. Matières dentaires polymérisables contenant de 20 à 65% en poids de monomères polymérisables habituellement utilisés dans ce domaine, qui présentent au moins une double liaison apte à la polymérisation radicalaire et de 10 à 60% en poids d'une matière de charge microporeuse choisie parmi $SiO_2$, $Al_2O_3$ ou le silicate de calcium, ainsi qu'éventuellement des additifs connus pour des matières dentaires, **caractérisées en ce que** la matière de charge présente :
   a) une granulométrie moyenne de 0,5-50 $\mu$m;
   b) une surface BET d'au moins 200 $m^2/g$ ;
   c) un volume de pores de 0,7-5 ml/g ; et
   d) un diamètre de pores de 10-50 nm.

2. Matières dentaires polymérisables selon la revendication 1, **caractérisée en ce que** la matière de charge est silanisée.

3. Matières dentaires polymérisables selon les revendications 1 et 2, **caractérisées en ce que** le monomère polymérisable est constitué d'un mélange de dérivés méthacryliques mono- et polyfonctionnels.

4. Matières dentaires polymérisables selon les revendications 1 à 3, **caractérisées en ce que** le monomère polymérisable contient du bis-GMA.

5. Utilisation de $SiO_2$, $Al_2O_3$ ou de silicate de calcium microporeux comme matières de charge, dans des matières dentaires polymérisables qui contiennent de 20 à 65% en poids d'un monomère polymérisable habituellement utilisé dans ce domaine, présentant au moins une double liaison apte à la polymérisation radicalaire, **caractérisée en ce que** les matières de charge présentent :
   a) une granulométrie moyenne de 0,5-50 $\mu$m ;
   b) une surface BET d'au moins 200 $m^2/g$ ;
   c) un volume de pores de 0,7-5 ml/g ; et
   d) un diamètre de pores de 10-50 nm.

6. Utilisation selon la revendication 5, dans des matériaux dentaires polymérisables, pour la mise en oeuvre de travaux odontologiques de restauration et de réparation.

7. Utilisation selon la revendication 6, dans des matières d'obturation dentaire, dans des matériaux

dentaires pour la fabrication de prothèses dentaires ou dans des matières destinées à des couronnes et à des ponts.